(19) Europäisches Patentamt
European Patent Office
Office européen des brevets

(11) Numéro de publication: **0 117 811**
**A2**

(12) # DEMANDE DE BREVET EUROPEEN

(21) Numéro de dépôt: **84400344.2**

(22) Date de dépôt: **20.02.84**

(51) Int. Cl.³: **A 61 K 47/00**, A 61 K 31/415

(30) Priorité: **23.02.83 FR 8302960**
**14.11.83 FR 8318055**

(43) Date de publication de la demande: **05.09.84**
**Bulletin 84/36**

(84) Etats contractants désignés: **AT BE CH DE FR GB IT LI LU NL SE**

(71) Demandeur: **SANOFI, société anonyme, 40, Avenue George V, F-75008 Paris (FR)**

(72) Inventeur: **Crisafulli, Emilio, Viale san Gimignano, 12, Milano (IT)**
Inventeur: **Sagon, Jean, 68, rue Jauffré Rudel La Paillade, F-34100 Montpellier (FR)**
Inventeur: **Mosse, Madeleine, La Chanterelle Chemin du Réservoir de Montmaur, F-34100 Montpellier (FR)**

(74) Mandataire: **Gillard, Marie-Louise et al, Cabinet Beau de Loménie 55, Rue d'Amsterdam, F-75008 Paris (FR)**

(54) **Compositions pharmaceutiques antifongiques utilisables par voie orale.**

(57) L'invention concerne des compositions pharmaceutiques à action antifongique pour administration orale.

Elles contiennent à titre d'ingrédient actif un dérivé imidazolé de formule I:

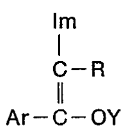

(I)

dans laquelle Ar représente un groupe phényle, naphtyle ou un radical hétérocyclique, R est un groupe alkyle droit ou ramifié, cycloalkyle saturé ou non, éventuellement substitué par un groupe aryle ou aralkyle; Im représente un groupe 1 H-imidazol-1-yle éventuellement substitué; Y est un groupe alkyle droit ou ramifié cycloalkyle saturé ou non éventuellement substitué par un groupe aryle ou aralkyle; lorsque Y représente une chaîne alkyle, celle-ci peut être interrompue une ou deux fois par des groupes $-O-$, $-S-$, $-SO-$, $-SO_2-$, en combinaison avec un excipient pharmaceutiquement acceptable choisi parmi les dérivés de polyalcools.

EP 0 117 811 A2

Compositions pharmaceutiques antifongiques utilisables par voie orale.

La présente invention concerne des compositions pharmaceutiques à action antifongique pour l'administration orale contenant un dérivé imidazolé.

La demande de brevet européen No. 8804 concerne une famille de composés dérivés de l'imidazole présentant des propriétés antifongiques et répondant à la formule générale :

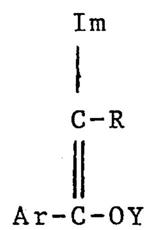

(I)

dans laquelle

- Ar représente un groupe phényle ou naphtyle ou encore un radical hétérocyclique ayant un ou deux cycles pouvant éventuellement être mono ou polysubstitué.
- R désigne l'hydrogène, un groupe alkyle droit ou ramifié, ou cycloalkyle, saturé ou non, éventuellement substitué par un groupe aryle ou aralkyle;
- Im représente un groupe 1H-imidazol-1-yle éventuellement mono-, di- ou tri-substitué;
- Y désigne un groupe alkyle droit ou ramifié ou cycloalkyle saturé ou non, éventuellement substitué par un groupe aryle ou aralkyle.

Lorsque Y représente une chaîne alkyle, celle-ci peut être interrompue une ou deux fois par des groupes -O-, -S-, -SO- ou $-SO_2-$.

Ces composés peuvent exister sous forme de base ou encore sous forme de sels, le nitrate étant le seul sel exemplifié.

Parmi cette famille, le (dichloro-2,4 phényl)-1 /(chloro-4 phénoxy)-2 éthoxy/-1 (imidazolyl-1)-2 propène, de formule

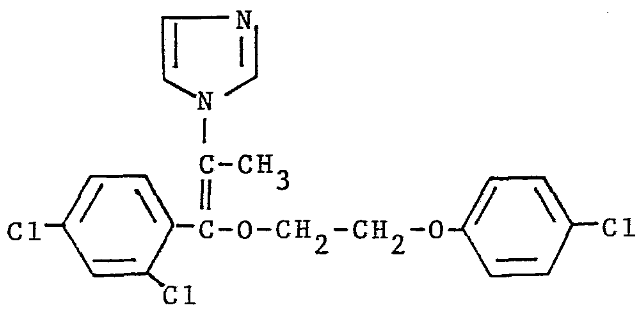

qui a reçu la Dénomination Commune Internationale "omoconazole", a été particulièrement étudié et s'est montré particulièrement intéressant, réunissant une activité antifongique et une activité antibactérienne sur certaines souches, telles que streptocoques et staphylocoques.

Toutefois les composés (I) sous forme de base ou de nitrate, s'ils présentent une activité intéressante par administration locale, se prêtent très mal à l'administration par voie générale (administration orale) par suite d'une très faible biodisponibilité.

Plus particulièrement, il n'est pas possible d'utiliser les composés de formule I ci-dessus, notamment l'omoconazole, par voie orale car on ne réussit pas à atteindre des taux sanguins thérapeutiquement actifs après administration orale de doses raisonnables desdits composés.

La demande de brevet allemand publiée sous le No. 3 010 041 décrit des médicaments à base de dérivés benzimidazolés qui contiennent lesdits benzimidazoles, notamment le mébendazole, en mélange avec un phospholipide. Selon ladite demande de brevet, des médicaments déjà utilisés par voie orale sont mélangés aux phospholipides pour obtenir une absorption améliorée ainsi qu'une diminution des effets secondaires.

Il est également connu que la lécithine, notamment la lécithine d'oeuf, inhibe, in vitro, l'activité antimycotique du chlotrimazole et du miconazole (Antimicrobial Agents Chemotherapy 1978, 13, 423-426).

On a maintenant trouvé d'une façon surprenante que les composés de formule I ci-dessus, notamment l'omoconazole, montrent une excellente activité orale lorsqu'ils sont administrés aux mammifères en mélange avec la lécithine.

On a aussi trouvé qu'on obtient une excellente activité orale lorsqu'on administre à un mammifère un composé de formule I, notamment l'omoconazole ou un de ses sels pharmaceutiquement acceptables, en mélange avec un dérivé polyalcool.

L'objet de la présente invention est donc de fournir des compositions pharmaceutiques contenant un composé de formule (I), utilisables par la voie orale pour le traitement par voie générale des affections mycotiques.

Ces compositions pharmaceutiques à action antifongique orale comprennent en tant qu'ingrédient actif un composé de formule générale (I) ou un de ses sels pharmaceutiquement acceptables, en mélange avec un excipient pharmaceutiquement acceptable, contenant un dérivé de polyalcool choisi parmi le groupe consistant en :

. les polyalcools eux-mêmes ;
. les mono-, di- ou tri-esters desdits polyalcools ;
. les polyéthers desdits polyalcools ou en leur mélange.

Le terme "dérivé polyalcool", tel qu'utilisé ici, désigne des dérivés de polyalcools pharmaceutiquement

acceptables ayant un poids moléculaire compris entre 150 et 2000, plus particulièrement des composés ayant plus d'un hydroxyle dans leurs molécules ainsi que leurs éthers ou esters, éventuellement sous forme polymérisée.

Les dérivés polyalcool comprennent par exemple les alcools dérivés des sucres,tels que le sorbitol, le mannitol et similaires, leurs esters, tels que le sorbitane monolaurate, le sorbitane monooléate,le sorbitane monopalmitate, le sorbitane monostéarate, le sorbitane trioléate ou le sorbitane tristéarate.

Les dérivés polyalcool comprennent aussi des esters de glycols inférieurs, tels que le propylèneglycol monostéarate et le propylèneglycol monooléate.

Les dérivés polyalcool comprennent aussi des polymères d'alcools inférieurs, tels que l'alcool polyvinylique et les polymères de glycols inférieurs, tels que les polyéthylèneglycols et les polypropylène-glycols et le polyoxyl 40 stéarate.

Les dérivés polyalcool comprennent aussi les esters du glycérol avec les acides gras, à savoir des monoglycérides, des diglycérides ou des triglycérides, tels que l'oléine, la palmitine, ainsi que leurs mélanges, tels que la lécithine.

Les dérivés polyalcool comprennent aussi les mélanges de glycérides qui forment les huiles naturelles, telles que l'huile de blé, l'huile d'olive,l'huile de grains de lin, l'huile de grains de tournesol, l'huile de palme, l'huile d'arachide, l'huile de soja, l'huile de sésame et similaires.

Les dérivés polyalcool comprennent aussi les huiles traitées avec du polyéthylèneglycol de façon à obtenir une transestérification partielle; les huiles ainsi traitées sont ici désignées "huiles polyoxyéthy-lénées".

Les dérivés polyalcool comprennent enfin les mélanges de glycérides et de polyglycols partiellement

estérifiés par des acides gras ayant de 6 à 18 atomes de carbone.

Les compositions pharmaceutiques ainsi obtenues peuvent se présenter sous la forme de solution, de solu-suspension, de coprécipité ou de cofondu. Eventuellement, elles peuvent être diluées avec des solvants utilisables en pharmacie et en particulier avec des alcools, notamment avec de l'éthanol.

Ces compositions pharmaceutiques peuvent éventuellement être additionnées de substances destinées à en modifier les caractéristiques physiques. Par exemple, les solutions peuvent être gélifiées notamment par addition de dioxyde de silicium colloïdal.

Enfin les compositions pharmaceutiques selon l'invention peuvent être additionnées de substances destinées à améliorer leurs propriétés gustatives et notamment d'agents édulcorants ou aromatisants ainsi qu'il est connu de l'homme de l'art.

Les compositions pharmaceutiques selon l'invention peuvent contenir de 5 à 15% de principe actif (I), environ 10% de préférence, et elles peuvent être utilisées, suivant le cas, sous forme de solutions, sous forme de capsules molles de gélatine ou encore sous forme de gélules ou de comprimés.

Pour le traitement des mycoses, la posologie varie largement suivant la nature et la gravité de l'affection à traiter. Le plus souvent chez l'adulte, par voie orale, elle est comprise entre 0,2 et 3 g de principe actif par jour, répartie en plusieurs prises. Le principe actif préférentiel est l'omoconazole.

Les exemples suivants non limitatifs permettront de mieux comprendre la portée de l'invention.

EXEMPLE 1 :

Solution à 10% de nitrate d'omoconazole.

On dissout par agitation à température ordinaire 10 g de nitrate d'omoconazole dans 100 ml d'Emulmetik 146®.

L'Emulmetik 146 est un produit commercialisé par la Société Lecithos à base de lécithine et d'agent tensioactif.

EXEMPLE 2 :

Capsules molles de gélatine.

Selon le procédé Scherer, on remplit des capsules molles de gélatine avec 1 ml de la solution mentionnée ci-dessus, de façon à obtenir des capsules contenant 100 mg de nitrate d'omoconazole.

EXEMPLE 3 :

Gelules.

La solution huileuse solidifiée par addition de cire ou de dioxyde de silicium est introduite dans des gélules de façon à contenir 25 ou 50 mg de principe actif.

EXEMPLE 4 :

On place dans un récipient fermé hermétiquement 100 g d'omoconazole, 90 g de monooléate de sorbitane polyoxyéthylené à 20 moles d'oxyde d'éthylène, 720 g de lécithine et 90 g d'éthanol.

On chauffe le mélange à 40°C sous agitation pendant 48 heures.

Après refroidissement, on obtient une solution contenant 10% d'omoconazole.

EXEMPLE 5 :

On agite à 60° C pendant 30 minutes le mélange de 100 g d'omoconazole et 900 g de polyéthylèneglycol 300.

Après refroidissement, on obtient une solution contenant 10% d'omoconazole.

EXEMPLE 6 :

On agite à température ambiante pendant 48 heures 100 g d'omoconazole et 900 g d'huile de ricin polyoxyéthylénée à 33 moles d'oxyde d'éthylène. On obtient ainsi une solu-suspension contenant 10 % d'omoconazole.

EXEMPLE 7 :

On agite à température ambiante pendant 48 heures 100 g d'omoconazole, 600 g d'huile de ricin polyoxyéthylénée à 33 moles d'oxyde d'éthylène et 300 g de polyéthylèneglycol 300.

On obtient ainsi une solu-suspension contenant 10 % d'omoconazole.

EXEMPLE 8 :

On ajoute sous agitation 50 g d'omoconazole à 50 g de sorbitol fondu préalablement. On obtient ainsi une pâte blanchâtre qu'on coule sur un support en verre et qu'on sèche à l'étuve à 50° C pendant 24 heures.

Après raclage, on broye le solide et on calibre les particules entre 0,16 et 0,50 mm.

EXEMPLE 9 :

On dissout 1,5 g de monooléate de sorbitane polyoxyéthyléné à 20 moles d'oxyde d'éthylène dans 600 ml d'alcool éthylique à 80 % par volume chauffé à 50°C. On ajoute 70 g de sorbitol sous agitation puis 30 g d'omoconazole et poursuit l'agitation pendant 2 heures jusqu'à dissolution totale. On évapore la solution sous courant d'air tiède (40 à 50°C) puis on sèche le coprécipité à l'étuve à 50°C.

On broye le solide et calibre les particules entre 0,16 et 0,500 mm.

EXEMPLE 10 : Sachets

La solution obtenue dans les exemples 4 ou 5 est gélifiée par addition de 5% de dioxyde de silicium colloïdal puis conditionnée dans des sachets contenant

5,05 g de préparation, soit 0,5 g d'omoconazole.

EXEMPLE 11 : Capsules molles

La solution obtenue dans les exemples 4 ou 5 ou la solution suspension des exemples 3 ou 4 peut être utilisée pour remplir des capsules molles selon le procédé Scherer, chaque capsule contenant 0,1 g d'omoconazole.

EXEMPLE 12 : Gélules

A la dispersion obtenue selon l'exemple 8, on ajoute 1 % de dioxyde de silicium et on remplit des gélules contenant 100 mg d'omoconazole.

EXEMPLE 13 : Comprimés

On prépare de façon habituelle des comprimés contenant :

. Coprécipité d'omoconazole (selon exemple 9).. 169,2 mg
. Lactose microcristallin........................ 90,8 mg
. Avicel pH 101................................. 131,2 mg
. Carboxyméthylamidon sodique.................. 4,0 mg
. Stéarate de magnésium ........................ 4,0 mg
. Dioxyde de silicium colloïdal................ 0,8 mg

On obtient ainsi un comprimé nu de 400 mg contenant 50 mg d'omoconazole.

EXEMPLE 14 : Gélules

Dans un mélangeur agitateur de liquide on porte progressivement à 40-45°C 100 g de gélucire 42/12 jusqu'à liquéfaction.

En maintenant à cette température, on introduit en pluie 50 g d'omoconazole finement broyé et on poursuit l'agitation jusqu'à homogénéisation complète.

On remplit alors des gélules No. 1 contenant 300 mg du mélange, soit 100 mg de principe actif.

Biodisponibilité des compositions selon l'invention

La biodisponibilité des compositions pharmaceutiques selon l'invention a été étudiée chez le babouin en comparaison avec le produit (I) seul.

Les études ont été conduites à la dose de 100 mg/kg d'omoconazole et les produits ont été administrés aux singes à jeun par sonde gastrique. Des prélèvements sanguins ont été réalisés aux temps 0,5 - 1 - 1,5 - 2 - 2,5 - 3 - 4 - 5 - 6 - 8 - 10 et 24 heures après l'administration orale. Les dosages des taux plasmatiques d'omoconazole ont été effectués par une méthode microbiologique réalisée à l'aide d'une technique de diffusion en milieu gélosé avec comme germe test Candida macedoniensis.

Les résultats suivants qui ont été obtenus représentent les valeurs moyennes déterminées sur des lots de 3 animaux /figure 1: avec la formulation de l'exemple 1 (courbe o—o), avec l'omoconazole seul (courbe •——•)./

Sur ces courbes, on peut constater que le pic plasmatique (ou concentration maximale) obtenu pour la solution selon l'invention est de 10,95 µg/ml alors qu'il n'est que de 1,35 µg/ml pour la suspension aqueuse.

De même l'aire sous la courbe est de 116 µg x h/ml pour la solution de l'invention alors qu'elle n'est que de 11,5 µg x h/ml pour la suspension aqueuse.

On peut en déduire que la solution selon l'invention améliore d'un facteur de l'ordre de 10 la pharmacocinétique de l'omoconazole en suspension aqueuse.

Des essais ont été effectués selon la même méthode, avec les formulations des exemples 4, 5, 6, 9 et 14. Pour les 3 premiers cas, les formulations ont été administrées telles quelles (solution) tandis que les compositions des exemples 9 et 14 et l'omoconazole ont été administrés dans des gélules No. 2.

Le tableau 1 ci-après rassemble les valeurs moyennes, obtenues sur 4 animaux, des paramètres pharmacocinétiques obtenus avec les 5 formulations selon l'invention ainsi qu'avec l'omoconazole.

TABLEAU 1

| Formulation | Concentration maximale mg/l | Temps de C. Max. en heures | Aire sous la courbe mg/l. h |
|---|---|---|---|
| Omoconazole........ | 1,35 | 5,5 | 11,6 |
| Formulation Ex 4... | 5,4 | 7,8 | 57,3 |
| Formulation Ex 5... | 5,1 | 9 | 65 |
| Formulation Ex 6... | 9,6 | 7 | 89,8 |
| Formulation Ex 9... | 2,6 | 5,5 | 46,8 |
| Formulation Ex 14.. | 7 | 6,6 | 71,5 |

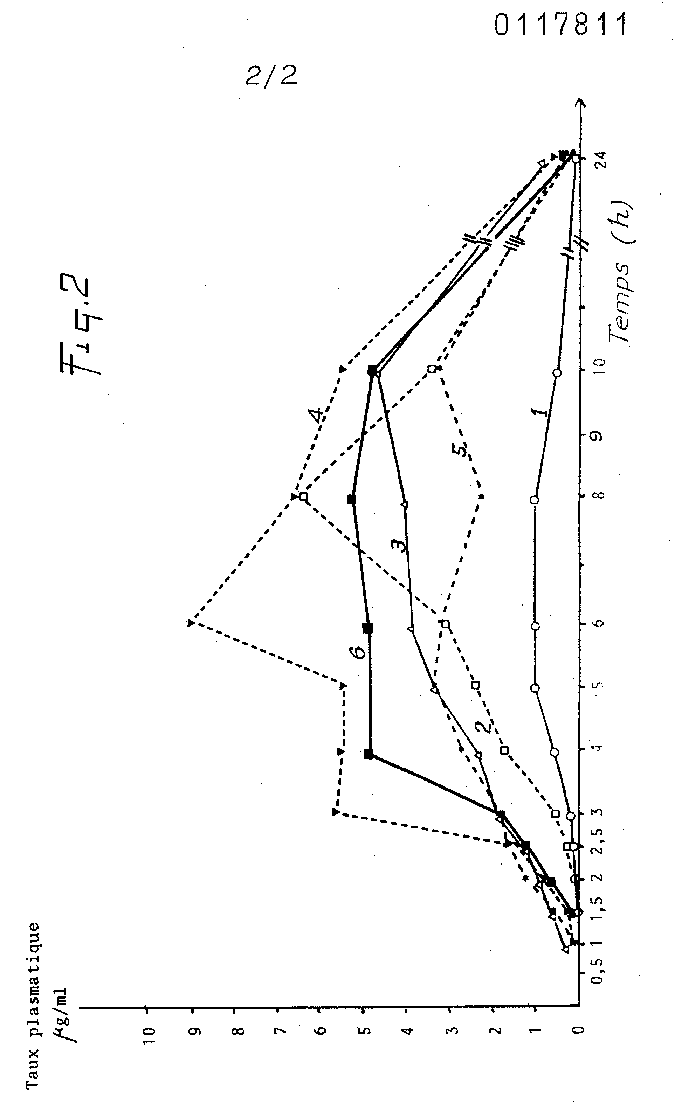

La figure 2 illustre les taux plasmatiques obtenus aux différents temps de prélèvements chez un des animaux soumis à l'expérimentation.

Les courbes de la figure 2 correspondent respectivement à :

- courbe 1 (tracé o—o) : Omoconazole matière première
- courbe 2 (tracé ◻—◻) : formulation de l'exemple 4
- courbe 3 (tracé △—△) : formulation de l'exemple 5
- courbe 4 (tracé ▼—▼) : formulation de l'exemple 6
- courbe 5 (tracé ✦—✦) : formulation de l'exemple 9
- courbe 6 (tracé ■—■) : formulation de l'exemple 14

On constate que la concentration maximale obtenue est augmentée de 2 à 7 fois selon les formulations étudiées et surtout que l'aire sous la courbe est augmentée d'un facteur variant de 4 à 8 selon les cas.

## REVENDICATIONS

1. Composition pharmaceutique antifongique utilisable par voie orale, caractérisée en ce qu'elle contient, à titre d'ingrédient actif, un dérivé imidazolé de formule :

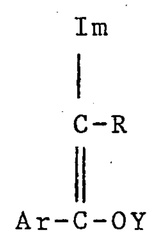

(I)

dans laquelle :

- Ar représente un groupe phényle ou naphtyle ou encore un radical hétérocyclique ayant un ou deux cycles pouvant éventuellement être mono- ou polysubstitué ;
- R désigne l'hydrogène, un groupe alkyle droit ou ramifié, ou cycloalkyle, saturé ou non, éventuellement substitué par un groupe aryle ou aralkyle ;
- Im représente un groupe 1H-imidazol-1-yle éventuellement mono-, di- ou tri-substitué ;
- Y désigne un groupe alkyle droit ou ramifié ou cycloalkyle saturé ou non, éventuellement substitué par un groupe aryle ou aralkyle.

Lorsque Y représente une chaîne alkyle, celle-ci peut être interrompue une ou deux fois par des groupes -O-, -S-, -SO- ou $-SO_2-$, ou un de ses sels pharmaceutiquement acceptables, en combinaison avec un excipient pharmaceutiquement acceptable, contenant un dérivé de polyalcool choisi parmi les polyalcools, les mono-, di- ou tri-esters desdits polyalcools, les polyéthers desdits polyalcools ou leurs mélanges, ledit dérivé ayant un poids moléculaire compris entre 150 et 2000.

2. Composition selon la revendication 1, caractérisée en ce que le dérivé imidazolé est le (dichloro-2,4 phényl)-1 [(chloro-4 phénoxy)-2 éthoxy]-1 (imidazolyl-1)-2 propène ou l'un de ses sels pharmaceutiquement acceptables.

3. Composition selon l'une des revendications 1 ou 2, caractérisée en ce que le dérivé imidazolé est sous la forme de nitrate.

4. Composition selon l'une quelconque des revendications 1 à 3, caractérisée en ce que le dérivé polyalcool est du sorbitol, du monooléate de sorbitane polyoxyéthyléné, de la lécithine du polyéthylèneglycol 300, de l'huile de ricin polyoxyéthylénée ou un mélange d'huile de ricin polyoxyéthylénée et de polyéthylèneglycol 300.

5. Composition selon l'une quelconque des revendications 1 à 3, caractérisée en ce que l'excipient est de la lécithine.

6. Composition selon l'une quelconque des revendications 1 à 3, caractérisée en ce que l'excipient est un mélange de lécithine et de monooléate de sorbitane polyoxyéthyléné.

7. Composition selon l'une quelconque des revendications 1 à 6, caractérisée en ce qu'elle contient de 5 à 15% de dérivé imidazolé.

8. Composition selon l'une quelconque des revendications 1 à 7, caractérisée en ce qu'elle est sous la forme de solutions, de capsules molles, de gélules ou de comprimés.

0117811

1/2

Fig. 1

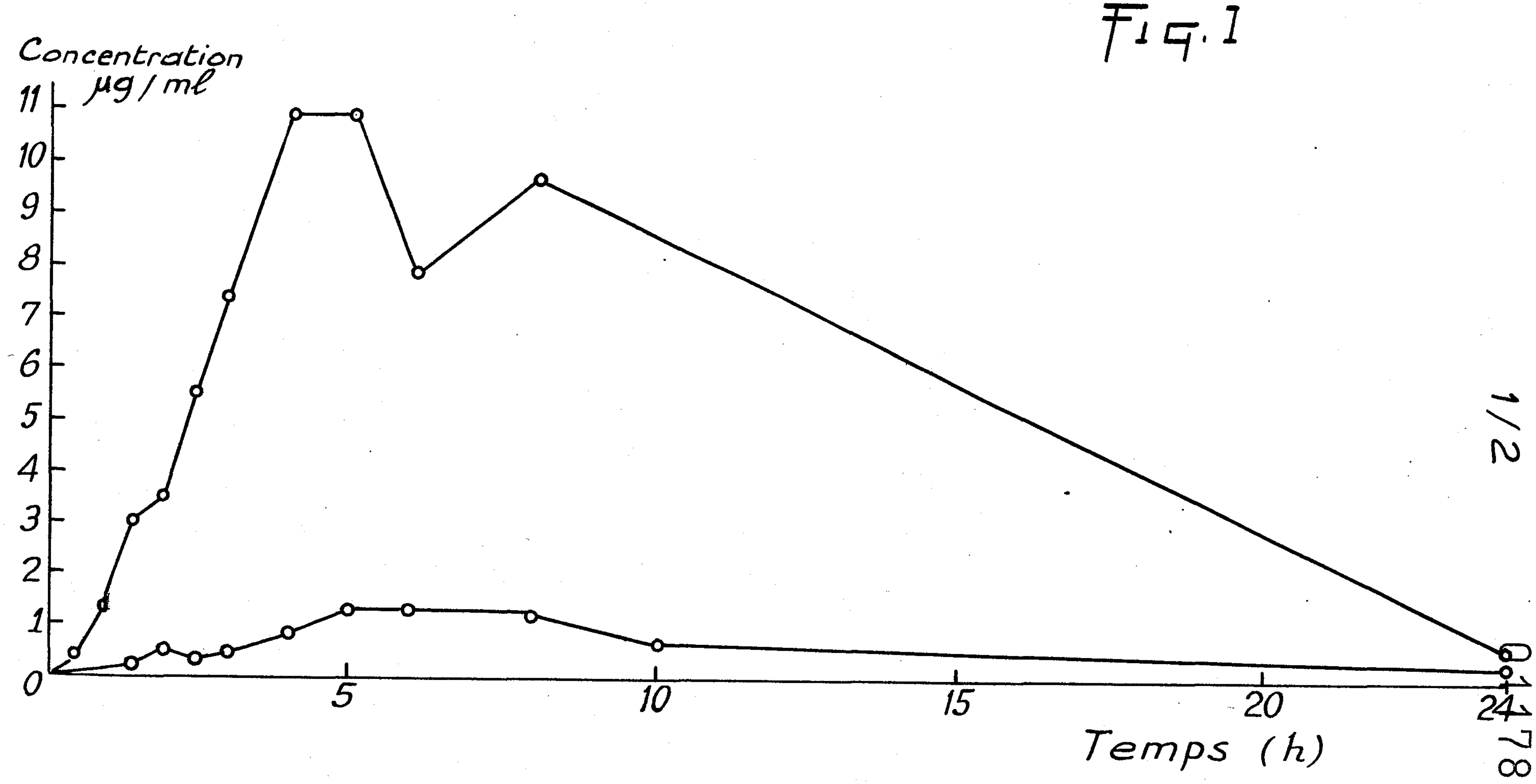

0117811

2/2

Fig.2